(19) Europäisches Patentamt
European Patent Office
Office européen des brevets

(11) **EP 2 552 300 B1**

(12) **EUROPEAN PATENT SPECIFICATION**

(45) Date of publication and mention
of the grant of the patent:
**14.10.2020 Bulletin 2020/42**

(21) Application number: **11718157.8**

(22) Date of filing: **16.03.2011**

(51) Int Cl.:
**A61B 5/00** *(2006.01)* **A61B 5/026** *(2006.01)*

(86) International application number:
**PCT/IB2011/051098**

(87) International publication number:
**WO 2011/117779 (29.09.2011 Gazette 2011/39)**

(54) **OPTICAL COHERENT IMAGING MEDICAL DEVICE**

MEDIZINISCHE VORRICHTUNG MIT KOHÄRENTER OPTISCHER BILDGEBUNG

DISPOSITIF MÉDICAL D'IMAGERIE PAR COHÉRENCE OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2010 PCT/IB2010/051332**

(43) Date of publication of application:
**06.02.2013 Bulletin 2013/06**

(73) Proprietor: **Aïmago S.A.**
**1015 Lausanne (CH)**

(72) Inventors:
• **ANDRE, Marc**
**3095 Spiegel b. Bern (CH)**
• **FRIEDRICH, Michael**
**1800 Vevey (CH)**
• **NAUMENKO, Andrey**
**1024 Ecublens (CH)**

• **FARKAS, Romain**
**1008 Lausanne (CH)**
• **LASSER, Theo**
**1026 Denges (CH)**

(74) Representative: **Grosfillier, Philippe**
**ANDRE ROLAND SA**
**Chemin des Charmettes 9**
**P.O. Box 5107**
**1002 Lausanne (CH)**

(56) References cited:
**EP-A1- 1 210 910          EP-A1- 1 210 910**
**DE-A1-102008 017 390   US-A1- 2007 100 245**
**US-A1- 2007 100 245    US-A1- 2007 100 245**
**US-B1- 6 178 340          US-B1- 6 178 340**
**US-B1- 6 178 340          US-B1- 7 113 817**
**US-B1- 7 113 817          US-B1- 7 113 817**

**Description**

**Field of invention**

The invention relates to an optical coherence imaging (OCI) medical imaging device.

**Definitions**

The following terms and related definitions are used in the present text.
"camera unit" is the physical unit where, as presented in this invention, the visualization screen and the OCI optics, including OCI sensor, illumination and other optics are implemented. This unit usually is moved as a whole.
"hand-movable camera" is a camera unit that can be moved by the user with little force. Usually the hand-movable camera is mounted to a supporting arm.
"OCI map" is any digital two or three dimensional data that is extracted from the optical coherent imaging. An example is the perfusion extracted from LDI. For 2-dimensional data it can be used directly as an image with x-y coordinates. 3-dimensional data can be reduced to 2D using projection or slicing. The OCI map as used in this document can also be a fOCI map.
"fOCI map" is a functional imaging result as disclosed for instance in WO 2010/004364. This definition matches the definition given in that patent application.
"white-light image" is any still or video image created with the use of an image sensor (CMOS, CCD or other) and being displayed as such, thus without further intensive processing other than filtering and improving the image quality. The image can either be color or black/white. As an example: all digital camera and video equipment produce a white-light image. The term white-light was introduced to show the difference to an optical coherent image or OCI map. It shall not be limited to pure white-light illumination but also include image from other color light source that was taken as standard photo.
"real-time" applied to visualization is a reasonable visualization update frequency with short latency so that the user can have an immediate feedback from any change of the observed area or the movement of the camera.
"confidence level" is any calculated or measured characteristic of an OCI map that summarizes the confidence with regard to the correct OCI map value for a given pixel, for a given region or for the overall OCI map. Example factors that can be reflected in the confidence level are imaging sharpness, curvature effects, camera stability, and noise.
"display" and "screen" shall be used as synonyms.
"aperture" shall be referenced as the entry/exit point(s) of the OCI optical path to the camera unit.

**State of the art**

There exists a number of imaging techniques in the medical field. X-ray based imaging, MRI, ultrasound imaging are proven techniques that are commonly used. Another group of imaging techniques is Optical Coherent Imaging (OCI).
Optical coherent imaging is a non-contact imaging utilizing, to a large extent, the physical properties and in particular the coherence properties of light. This imaging modality integrates a detector technology, combined with an appropriate coherent light source and an image processing unit for extracting the flow characteristics of the observed body area of interest. Thereby, it allows the diagnosis or observation of multiple diseases and disorders such as peripheral vascular diseases, skin irritations, diabetes, burns, organ transplants, tissue grafts and even functional brain imaging. This method is non-invasive because it involves no physical contact; therefore risk of infection and discomfort are greatly avoided.
Sub-classes of OCI include, but are not limited to: Laser Doppler Imaging (LDI), Laser Doppler Spectroscopic Imaging (LDSI), Laser Speckle Imaging (LSI), Optical Coherence Tomography (OCT), Functional Optical Coherent Imaging (fOCI).
Existing OCI medical devices, like many other medical imaging tools, are at least partially static. The OCI sensors may be movable but the screen is permanently fixed to a support.
It would be therefore convenient for the user, in one single movement, to simultaneously orient the OCI medical device on an observed area and view this area.
US7113817 discloses devices and methods provided for noninvasive and noncontact real-time measurements of tissue blood velocity. A digital imaging device such as a detector array is used that allows independent intensity measurements at each pixel to capture images of laser speckle patterns on any surfaces, such as tissue surfaces. The laser speckle is generated by illuminating the surface of interest with an expanded beam from a laser source such as a laser diode or a HeNe laser as long as the detector can detect that particular laser radiation. Digitized speckle images are analyzed using algorithms for tissue optics and blood optics employing multiple scattering analysis and laser Doppler velocimetry analysis. The resultant two-dimensional images can be displayed on a color monitor and superimposed on images of the tissues.

US2007100245 provides a laser blood flow imaging apparatus whose configuration is simple and which has functions of arithmetically and concurrently processing blood flows of all points of measurement. The laser blood flow imaging apparatus includes: a laser light irradiating unit for irradiating laser light of a predetermined wavelength to a blood flow within a vital tissue; an image capturing unit for capturing scattered light from the blood flow irradiated with the laser light; an image processing unit for processing an image of the captured blood flow per pixel; a value-of-blood flow calculating unit for calculating a value of blood flow based on data per the pixel and for processing as a color discernible image corresponding to the calculated value; and a display unit for displaying the image of the blood flows processed by the value-of-blood flow calculating unit as a two-dimensional color image.

US6178340 discloses a novel three-dimensional infrared imager for study of the vascular network so as to cause blood vessels to be visualized for accurate subcutaneous puncture during insertion of a hypodermic needle. Used are the properties of near infrared light as it is absorbed and reflected by the human body, allowing the user to visualize the tissues a few millimeters in depth from the skin's surface. The use of infrared light permits the manipulation of the acquired information to a grade of sensed visualization, which is impossible to reach with visible light. The human three-dimensional perception is imitated by use of a stereoscopic infrared viewer, and is designed to be user friendly, allowing the health care professional to work in his usual manner. A double image is superimposed on the viewer and the user wears blue-red eyeglasses to create the three-dimensional image from the double image on the viewer. With the depth perception provided by the three-dimensional image, the user can accurately penetrate the vein on the initial attempt, thereby lowering the fear of venous punture and causing the patient to be at ease, while lowering the incidence of medical complications associated with inaccurate puncture. A liquid crystal display (LCD) mounted in a frame is provided on the upper surface of the apparatus. Sensitive, charge-coupled devices are provided which develop images and deliver them to a microprocessor which synchronizes the images and allows the user to manipulate the images as desired, controlling effects such as brightness, contrast, sharpness and edge enhancement. The imaging system incorporates the use of a contrast agent to enhance the image and allow for study of the vascular system. The imaging system incorporates the use of a coherent source of light, such as an infrared laser.

**General description of the invention**

The present invention provides a solution to the problem mentioned in the previous chapter.

It concerns an OCI medical imaging device according to claim 1.

Other advantageous features can be found in the dependent claims.

Advantageously the device is a hand-movable camera unit featuring a display that works as a virtual window. The display allows for the real-time visualization of OCI. The display also allows for the OCI-enhanced real sight visualization. Handling of the device is very natural to the user because he just moves the virtual window to the region of interest and sees on the screen the OCI map. If the device was fully transparent he would see the same region with direct eye view.

One of advantages of the invention is to provide a so far unachievable improvement in usability of an OCI medical device. It comprises OCI acquisition tools and visualization display within a single, preferably hand-movable housing. The invention also provides other innovative improvements that will be discussed later in this document.

The invention also reduces the learning time for the user significantly. The user can move the virtual window over the patient. In this window the user doesn't just see a picture of the surface but also a real-time visualization of body parameters. The body parameter can be blood perfusion, blood concentration, blood speed, any fOCI map or other body functions of interest taken with the technique of Optical Coherent Imaging.

The invention not only provides a handheld but also a hand-movable device. This considerably facilitates the user's work. By "hand-movable" it is meant a mobile device that can be moved by hands during its use but that, unlike a handheld device, can have some kind of support (e.g. a stand with a mechanical arm, a ceiling or wall mounted suspension, or some other). Such a support holds its corresponding hand-movable device while the device is used. Thus with the present invention users do not need to hold the device while using it, having their hands free for other manipulations.

The invention offers in particular the following advantages with regard to the existing OCI systems :

- Easy handling and focusing of the device;
- Shorter learning time to use the device;
- Overlooking the area of interest with a direct eye view and with an OCI-enhanced view simultaneously, with a user having free hands available for other manipulations at the same time;
- No dead angles while viewing the area of interest

## Detailed description of the invention

The invention will be better understood through non-limitative examples that are illustrated by the following figures:

Figure 1 shows a side view of a proposed Embedded OCI system.

Figure 2 shows pseudo perspective views of possible mounting of the screen to the camera

Figure 3 shows the simple focus laser indication system with 3 collimated light sources

Figure 4 screenshots of an Embedded OCI system with user interface rotation for working on two sides of the device

Figure 5 shows mixture map of an LDI perfusion flow over the white-light image.

Figure 6 shows an example of overlay over the OCI map with additional indications for the user.

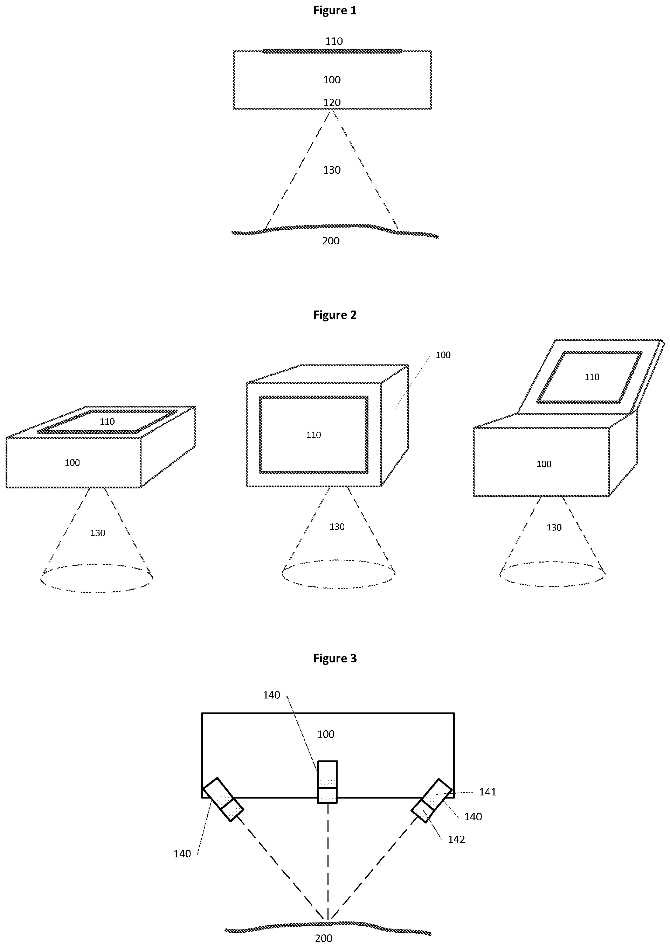

As shown on figure 1 the screen (110) is part of the same unit (camera unit, 100) as the OCI optics (120). If the user moves the camera unit he automatically also moves the screen for a similar distance. The OCI optics consists of laser(s), 2D light sensor(s) and possibly other optical and mechanical elements. A 2D light sensor is an image sensor of any technology that can work with visible, infrared or ultraviolet light. The OCI optics aperture (or 2D light sensor aperture) is the entry point of the optics path into the camera unit. In the best case the screen is on the opposite side of the aperture. The OCI optics with its optics path (130) visualizes a body parameter of the observed area of the patient (200). In addition a standard white-light camera observes the same observed area, either through the same or a similar optical path. The size of the observed area for OCI and white-light can be different.

For the completeness it shall be mentioned that the screen can also be attached on other positions of the camera. Possible positions are the side or any other angle. Figure 2 shows some examples. For the usability reasons mentioned in the background discussion, in most cases the camera unit is also mounted to a supporting arm. In that case the screen may also be mounted to that arm as long as it is in close proximity of the OCI optics and moves together with the OCI optics.

A processing unit further processes the data obtained from the OCI sensor and calculates the OCI maps. There are different processing techniques available to people skilled in the art. Processing is done using CPU, DSP, FPGA, ASIC or a combination of those. The processing unit can either be a part of the camera unit or a separate unit with a wired or a wireless connection.

On the display an OCI map, a white light image or a combination of both is shown in real-time. The orientation of the image matches the orientation of the visualized object. This means that the user sees an object in the same orientation on the screen as he would see it without the screen with a direct eye view. In optimal case the observed surface is shown in a 1:1 scaling thus the image on the screen has roughly the size of the observed area, but the scaling can be different without limiting the invention.

OCI is a non-contact technology and has to work at some reasonable working distance of a few centimeters up to a meter, while the best working distance for usability reasons ranges from 15 cm to 30 cm. In order to find the focus a simple focus indication system can be implemented. A pattern is projected to the observed skin area which has a distance depending scale and/or shape. This pattern indicates if the camera is within working distance. In addition it is also possible to show if the camera is too close or too far with regard to the focal distance.

A possible implementation of this focus indication system is shown in Figure 3. At least 2 collimated light sources (140) consisting of a light source (141) and a collimating optics (142) are placed on different positions of the camera unit (100). These collimated light sources project a small point on the observed surface (200). The small points collide in the focus distance of the camera unit. In the best case the collision point is also in the center of the observed area. With this simple system the user has to bring the camera in such a distance that the two points are projected on the same spot on the observed area and thus only a single projected point is visible. When adding a 3rd laser it is also possible to show if the camera is too close or too far from the observed surface with regard to the focal distance. Because the projected triangular pattern changes its orientation when camera unit passes through its focal point. With good real-time characteristics of the OCI system this is not necessary, because the user determines with the dynamics of imaging if the camera unit is too far or too close.

A good color for the focus indication light is green because this is well seen on the skin. In addition most OCI systems use infrared lasers and infrared detectors. It is easy to filter the green focus indication light from being captured by the OCI detector using band-pass, long-pass filter, dichroic mirror or any other filter known to those skilled in the art.

The screen acts as HMI (human machine interface). Its primary function is to show an OCI map, a white light image or a mixture map. It is also possible to show multiple maps at the same time. In the best case the user can switch between different maps or map combinations. The secondary functions of the screen are: to allow users to configure the system and to show other information needed for functioning of the device.

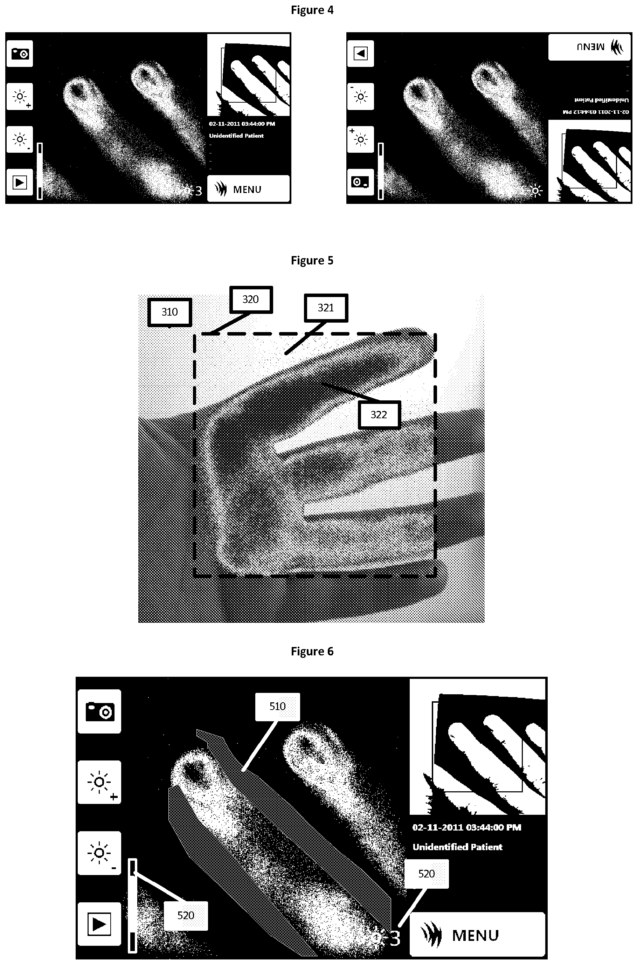

Especially in operating rooms an OCI device may be used by two doctors working on opposite sides of the patient. Another aspect of this invention is the rotation or swapping of the user interface. Rotating or swapping involves all user interface parts on the screen but it doesn't necessary mean that the user interface layout remains exactly the same. Figure 4 gives an example. In addition the OCI map and white-light image do not turn, because their orientation with regard to the user shall remain, as described in the paragraphs above. The layout may be differently designed in different orientations because of some external constraints. In the example given in Figure 4 this is the location of the buttons on the left, because the device has adjacent physical buttons.

The mixture map, as presented in this invention, is an overlay of an OCI map over the white-light image. In order to achieve a good image, irrelevant values of the OCI map can be removed. For perfusion these would be the non- or very low-perfused values. These values are made fully transparent while the others are shown with some transparency ($\alpha$). The transparency can either be a uniform fixed or a user-set uniform value, which does not form part of the invention. According to the invention, the transparency is calculated depending on the value (v) and/or the confidence level (y) of the OCI map, again optionally with some user-configurable level. Of course other approaches or extensions, like taking neighboring pixels into the formula, are possible within the scope of the invention as defined by the claims.

| Uniform transparency | Non-uniform transparency |
|---|---|
| $\alpha = C_{user}$ | $\alpha_{xy} = C_{user} * f(v_{xy}, \gamma_{xy})$; with $v_{xy}$ being the OCI map value at coordinate (x,y) |

Non-uniform transparency, extended

$$\alpha_{xy} = c_{user} * f\left(v_{1,all}, v_{2,all}, \ldots, \gamma_{1all}, \gamma_{2all}, \ldots x, y\right);$$

with

$v_{1,all}$ being all data of a OCI map and $v_{2,all}$ being all data of a different OCI map

Figure 5 shows an example implementation where the perfusion of a finger is shown. In this example the white-light image (310) with a larger observed area is overlayed with an OCI map (320, border not visible to user). The non- and low-perfused values are set to fully transparent (321) while the other values don't have any transparency (322).

The image can further be extended with additional overlay information consisting of text, figures or drawings. This can be, but not limited to, the highlighting of interesting spots, the labeling of values or settings (530), indicating color bar (520) or the cover of regions with low confidence level (510). Figure 6 shows an example.

The user interaction with the OCI system may be by touch screen, by physical buttons, by foot pedal or remote buttons or by any combination of those. The touch screen technology can be resistive, capacitive or any other technologies known to those skilled in the art.

In the preferred way the device is small such that the user can view the screen and the observed area with direct eye view simultaneously. For that reason the screen should have a good viewing angle.

It is also possible to draw features like outline of the observed skin area, regions with some OCI map values above or below a threshold, or other data mentioned above for overlay information directly to the skin of the patient. This drawing is done using light projection or laser illumination. It can be single or multi-color. In the best case the user can select to enable or to disable the projection. There are many projection technologies known to people skilled in the art.

## Claims

1. An optical coherence imaging, OCI, medical imaging device comprising the following elements:

   - OCI optics (120) including a coherent light source (140);
   - a 2D light sensor (100) configured to detect light received from a region of interest of a subject;
   - a white-light camera configured to observe the region of interest and to provide a white-light image;
   - a screen (110); and
   - a processing unit configured to:

     (i) process data obtained from the 2D light sensor,

(ii) calculate an OCI map of blood perfusion from said data;

wherein all said elements are included in a single movable unit (100)
**characterized in that** the processing unit is further configured to (iii) show, on the screen (110), a which is an overlay of the OCI map over the white-light image, of the region of interest; and **in that** the transparency of the OCI map in the mixture map is calculated depending on an OCI value and/or a confidence level of the OCI map.

2. An OCI medical device according to claim 1 wherein said single movable unit comprises a first face facing in a first direction and a second face facing in a second direction opposite to the first direction, said screen being located on said first face and said 2D light sensor and/or an aperture of the 2D light sensor being located on said second face.

3. An OCI medical device according to claim 1 wherein said single movable unit comprises a first face facing in a first direction and a second face facing in a second direction different from the first direction, said screen being located on said first face and said 2D light sensor and/or an aperture of the 2D light sensor being located on said second face.

4. An OCI medical device according to claim 1 wherein said single movable unit comprises a rotatable panel and a first face, said screen being located on said rotatable panel and said 2D light sensor and/or an aperture of the 2D light source being located on said first face.

5. An OCI medical device according to one of the previous claims comprising real-time OCI means which are adapted to show on said screen a real-time OCI of an observed area.

6. An OCI medical device according to one of the previous claims comprising real-time color or black-and-white visualization means which are adapted to show on screen a real-time regular view of the region of interest.

7. An OCI medical device according to claim 1, wherein the OCI map is a 2D OCI map

**Patentansprüche**

1. Optische Kohärenzbildgebungs- bzw. OCI(Optical Coherence Imaging)-Medizinbildgebungsvorrichtung, die die folgenden Elemente umfasst:

- eine OCI-Optik (120) einschließlich einer kohärenten Lichtquelle (140);
- einen 2D-Lichtsensor (100), der zum Detektieren von Licht ausgebildet ist, das von einem Gebiet von Interesse eines Subjekts empfangen wird;
- eine Weißlichtkamera, die zum Beobachten des Gebiets von Interessen und Bereitstellen eines Weißlichtbilds ausgebildet ist;
- einen Bildschirm (110); und
- eine Verarbeitungseinheit, die zu Folgendem ausgebildet ist:

(i) Verarbeiten von Daten, die von dem 2D-Lichtsensor erhalten werden,
(ii) Berechnen einer OCI-Karte einer Durchblutung aus den Daten;

wobei sämtliche Elemente in einer einzigen beweglichen Einheit (100) enthalten sind,
**dadurch gekennzeichnet, dass** die Verarbeitungseinheit ferner zum (iii) Zeigen, auf dem Bildschirm (110), einer Mischkarte, die eine Überlagerung der OCI-Karte über dem Weißlichtbild ist, des Gebiets von Interesse ausgebildet ist;
und dadurch, dass die Transparenz der OCI-Karte in der Mischkarte in Abhängigkeit von einem OCI-Wert und/oder einem Konfidenzniveau der OCI-Karte berechnet wird.

2. OCI-Medizinvorrichtung nach Anspruch 1, wobei die einzige bewegliche Einheit eine erste Fläche, die in eine erste Richtung zeigt, und eine zweite Fläche, die in eine zu der ersten Richtung entgegengesetzte zweite Richtung zeigt, umfasst, wobei sich der Bildschirm auf der ersten Fläche befindet und sich der 2D-Lichtsensor und/oder eine Öffnung des 2D-Lichtsensors auf der zweiten Fläche befindet.

3. OCI-Medizinvorrichtung nach Anspruch 1, wobei die einzige bewegliche Einheit eine erste Fläche, die in eine erste Richtung zeigt, und eine zweite Fläche, die in eine von der ersten Richtung verschiedene zweite Richtung zeigt, umfasst, wobei sich der Bildschirm auf der ersten Fläche befindet und sich der 2D-Lichtsensor und/oder eine Öffnung des 2D-Lichtsensors auf der zweiten Fläche befindet.

**4.** OCI-Medizinvorrichtung nach Anspruch 1, wobei die einzige bewegliche Einheit eine drehbare Platte und eine erste Fläche umfasst, wobei sich der Bildschirm auf der drehbaren Platte befindet und sich der 2D-Lichtsensor und/oder eine Öffnung der 2D-Lichtquelle auf der ersten Fläche befindet.

**5.** OCI-Medizinvorrichtung nach einem der vorhergehenden Ansprüche, die Echtzeit-OCI-Mittel umfasst, die zum Zeigen von Echtzeit-OCI eines beobachteten Bereichs auf dem Bildschirm eingerichtet sind.

**6.** OCI-Medizinvorrichtung nach einem der vorhergehenden Ansprüche, die Echtzeitfarb- oder -Schwarzweiß-Visualisierungsmittel umfasst, die zum Zeigen einer regulären Echtzeitansicht des Gebiets von Interesse eingerichtet sind.

**7.** OCI-Medizinvorrichtung nach Anspruch 1, wobei die OCI-Karte eine 2D-OCI-Karte ist.

## Revendications

**1.** Dispositif d'imagerie médicale pour imagerie par cohérence optique, ICO, comprenant les éléments suivants :

- une optique ICO (120) comprenant une source de lumière cohérente (140) ;
- un capteur de lumière 2D (100) configuré pour détecter une lumière reçue en provenance d'une région d'intérêt d'un patient ;
- une caméra à lumière blanche configurée pour observer la région d'intérêt et pour fournir une image en lumière blanche ;
- un écran (110) ; et
- une unité de traitement configurée pour :

(i) traiter des données à partir du capteur de lumière 2D,
(ii) calculer une carte ICO d'une perfusion sanguine à partir desdites données ;

dans lequel tous lesdits éléments sont inclus dans une unité mobile unique (100)
**caractérisé en ce que** l'unité de traitement est en outre configurée pour (iii) afficher, à l'écran (110), une carte de mélange, qui est une superposition de la carte ICO sur l'image en lumière blanche, de la région d'intérêt ; et **en ce que** la transparence de la carte ICO dans la carte de mélange est calculée en fonction d'une valeur d'ICO et/ou d'un niveau de confiance de la carte ICO.

**2.** Dispositif médical ICO selon la revendication 1, dans lequel ladite unité mobile unique comprend une première face tournée dans une première direction et une seconde face tournée dans une seconde direction opposée à la première direction, ledit écran étant situé sur ladite première face et ledit capteur de lumière 2D et/ou une ouverture du capteur de lumière 2D étant situés sur ladite seconde face.

**3.** Dispositif médical ICO selon la revendication 1, dans lequel ladite unité mobile unique comporte une première face tournée dans une première direction et une seconde face tournée dans une seconde direction différente de la première direction, ledit écran étant situé sur ladite première face et ledit capteur de lumière 2D et/ou une ouverture du capteur de lumière 2D étant situés sur ladite seconde face.

**4.** Dispositif médical ICO selon la revendication 1, dans lequel ladite unité mobile unique comporte un panneau rotatif et une première face, ledit écran étant situé sur ledit panneau rotatif et ledit capteur de lumière 2D et/ou une ouverture de la source de lumière 2D étant situés sur ladite première face.

**5.** Dispositif médical ICO selon l'une des revendications précédentes, comprenant des moyens ICO en temps réel qui sont conçus pour afficher sur ledit écran une ICO en temps réel d'une zone observée.

**6.** Dispositif médical ICO selon l'une des revendications précédentes, comprenant des moyens de visualisation en temps réel en couleur ou en noir et blanc qui sont conçus pour afficher à l'écran une vue normale en temps réel de la région d'intérêt.

**7.** Dispositif médical ICO selon la revendication 1, dans lequel la carte ICO est une carte ICO 2D.

**Figure 1**

110

100

120

130

200

**Figure 2**

100

110

110

100

110

130

100

130

100

130

**Figure 3**

140

100

140

141

140

142

140

200

Figure 4

Figure 5

Figure 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010004364 A
- US 7113817 B
- US 2007100245 A
- US 6178340 B